# DEMANDE DE BREVET EUROPEEN

(11) **EP 2 607 876 A1**
(43) Date de publication de la demande: **26.06.2013**
(21) Numéro de dépôt: 11194613.3
(22) Date de dépôt: 20.12.2011
(51) Int. Cl.: G01L 9/00, G01L 9/10, G01L 9/14, A61B 5/103

(54) **Semelle pour la mesure des pressions plantaires et dispositif de suivi des pressions plantaires**

(71) Demandeur: Rocklinger, Marc, 74380 Bonne (FR); Vacherand, Pascal, 74380 Lucinges (FR); Brönnimann, Frédéric, 1261 Le Vaud (CH)
(72) Inventeur: Rocklinger, Marc, 74380 Bonne (FR); Vacherand, Pascal, 74380 Lucinges (FR); Brönnimann, Frédéric, 1261 Le Vaud (CH)
(74) Mandataire: Bugnion Genève

(57) **Abrégé**

Semelle (1) pour chaussure munie d'un circuit électronique comprenant une pluralité de capteurs de pression (2) connectés à un module électronique (3), le module électronique comprenant des moyens d'alimentation (10) pour alimenter les capteurs de pression en énergie et des moyens de mesure (11) pour effectuer des mesures de la tension en sortie de chacun des capteurs de pression.

## Description

### DOMAINE TECHNIQUE DE L'INVENTION

La présente invention concerne le domaine des instruments de mesure. L'invention porte en particulier sur une semelle pour chaussure munie d'un circuit électronique comprenant une pluralité de capteurs de pression connectés à un module électronique, le module électronique comprenant des moyens d'alimentation pour alimenter les capteurs de pression en énergie et des moyens de mesure pour effectuer des mesures de la tension en sortie de chacun des capteurs de pression. L'invention porte également sur un dispositif de suivi des pressions plantaires.

### ETAT DE LA TECHNIQUE ANTERIEURE

Les personnes neuropathiques ou diabétiques, compte tenu de la perte de sensibilité induite par ces pathologies, ont un risque accru de développer des ulcères plantaires. En effet, la perte de sensibilité empêche ces personnes de se rendre compte de l'apparition de telles pathologies qui peuvent évoluer jusqu'à nécessiter une hospitalisation longue et couteuse. Ainsi, pour aider des personnes à prendre conscience des pressions plantaires induites par la marche, que ce soit à but thérapeutique ou à but sportif, des appareils de mesure des pressions plantaires existent mais ceux-ci peuvent s'avérer encombrants et peu pratiques. On trouve par exemple, dans le domaine des instruments d'assistance à la pratique de certains sports, des semelles munies de capteur de pression qui permettent de mesurer en temps réel les pressions appliquées sur la plante des pieds. Cependant, ces semelles incluent généralement des capteurs capacitifs, résistifs ou piezo-électriques et il apparaît que de tels capteurs, généralement constitués de matériaux en céramique (diélectriques) ou en alliages métalliques, sont fragiles ce qui à pour conséquence de limiter leur durée de vie. De tels capteurs se révèlent en réalité peu propices à être installés sur une semelle, en particulier sur une semelle qui peut être amenée à être utilisée dans un environnement thérapeutique. De plus, les quelques système de suivi des pressions plantaires de l'art antérieur comprennent généralement des semelles munies d'un circuit électronique comprenant un très grand nombre de capteurs de pression (p.ex : 500-1000) et l'électronique de traitement des signaux est souvent déportée au travers de câbles sur un appareillage qui peut être portable ou fixe. L'utilisation de tels systèmes n'est souvent pas pratique et le cout engendré par un si grand nombre de capteur est élevé.

### EXPOSÉ DE L'INVENTION

Un premier but de l'invention est de fournir une semelle pour la mesure des pressions plantaires dans le but de suivre les altérations de la marche afin de prévenir des pathologies du système orthopédique (pied cheville, genoux, jambe, hanche) telles que par exemple les ulcères plantaires rencontrés principalement chez des patients neuropathiques ou diabétiques. Le patient peut ainsi améliorer sa marche et contribuer ainsi à traiter différents pathologies du système orthopédique.

Un deuxième but de l'invention est de fournir une semelle munie de capteurs de pression plus solides et dont la durée de vie est accrue.

Un troisième but de l'invention est de fournir des capteurs de pression dont la sensibilité est accrue permettant ainsi une meilleure mesure de la pression.

Un quatrième but de l'invention est de fournir une semelle comprenant un nombre de capteurs aussi faible que possible de manière à limiter les coûts de fabrication de la semelle.

Un cinquième but de l'invention est de fournir un dispositif de suivi des pressions plantaires qui permette d'afficher en temps réel ou différé l'évolution des pressions plantaires et qui soit pratique à utiliser. Un tel dispositif peut aussi bien être utilisé dans un but thérapeutique mais également dans un environnement sportif pour améliorer la prise en compte des pressions plantaires et ainsi permettre d'optimiser la pratique de sports tels que la marche sportive, la course à pied, le golf, le cyclisme, etc.

Selon l'invention, la semelle pour chaussure est munie d'un circuit électronique comprenant une pluralité de capteurs de pression connectés à un module électronique, le module électronique comprenant des moyens d'alimentation pour alimenter les capteurs de pression en énergie, des moyens de mesure pour effectuer des mesures de la tension en sortie de chacun des capteurs de pression et les capteurs de pression sont des capteurs de pression inductifs.

Selon l'invention, le module électronique peut comprendre des moyens de calcul pour déterminer, en fonction des mesures de la tension en sortie de chacun des capteurs de pression effectuées par les moyens de mesure, la pression exercée en plusieurs points de la semelle.

Selon l'invention, le module électronique peut comprendre des moyens de communication pour émettre des signaux radiofréquences.

Selon l'invention, le module électronique peut comprendre un accéléromètre pour effectuer des mesures des mouvements du module électronique.

Selon l'invention, le module électronique peut comprendre un organe de commutation permettant, en utilisant les mesures des mouvements du module électronique effectuées par l'accéléromètre, de faire basculer le module électronique dans un état de veille dans lequel l'énergie consommée par le module électronique est réduite.

Selon l'invention, chacun des capteurs de pression peut comprendre, empilées les unes sur les autres, au moins une couche cible en un matériau électriquement conducteur, au moins une couche élastiquement déformable en un matériau compressible et une bobine.

Selon l'invention, la bobine peut comporter une première couche conductrice en un matériau électriquement conducteur, une deuxième couche conductrice en un matériau électriquement conducteur et une couche isolante en un matériau électriquement isolant, la première couche conductrice étant connectée électriquement à la deuxième couche conductrice et la couche isolante étant disposée entre la première couche conductrice et la deuxième couche conductrice.

Selon l'invention, les moyens de communication peuvent comprendre un connecteur et un logiciel pour connecter le module électronique à un récepteur au moyen d'un câble.

Selon l'invention, les moyens d'alimentation peuvent comprendre une batterie rechargeable.

Selon l'invention, le module électronique peut comprendre des moyens d'indication pour indiquer l'état de charge de la batterie.

Selon l'invention, les moyens d'alimentation peuvent comprendre un module de couplage inductif pour coupler le module électronique à un chargeur inductif.

Selon l'invention, les moyens d'alimentation peuvent comprendre un module de couplage filaire pour connecter le module électronique à un chargeur au moyen d'un câble.

Selon l'invention, la semelle peut comprendre entre 4 et 50 capteurs de pression.

Selon l'invention, le module électronique peut comprendre des moyens de stockage pour stocker des données.

Selon l'invention, la semelle peut comprendre une pluralité de trous traversants.

Selon l'invention, la semelle peut comprendre une languette sur laquelle est disposé le module électronique de sorte que celui-ci puisse être à l'extérieur d'une chaussure lorsque la semelle y est disposée.

Un dispositif de suivi des pressions plantaires selon l'invention comprend une ou deux semelles tel que décrit ci-dessus et des moyens logiciels pour présenter graphiquement sur l'interface graphique d'un récepteur, en utilisant des informations transmises par la ou les semelles, l'évolution des pressions plantaires exercées en plusieurs points de la ou des semelles.

Selon une variante de l'invention, la semelle pour chaussure est munie d'un circuit électronique comprenant une pluralité de capteurs de pression connectés à un module électronique, le module électronique comprenant des moyens d'alimentation pour alimenter les capteurs en énergie, des moyens de mesure pour effectuer des mesures de la tension en sortie de chacun des capteurs de pression et un accéléromètre pour effectuer des mesures des mouvements du module électronique.

### DESCRIPTION DES FIGURES

D'autres caractéristiques et avantages de l'invention ressortiront à la lecture de la description qui suit, en référence aux figures annexées, dans lesquelles :
- la figure 1 montre une semelle selon l'invention ;
- la figure 2 montre un schéma fonctionnel d'un module électronique d'une semelle selon l'invention ;
- la figure 3 montre schématiquement la structure d'un capteur de pression d'une semelle selon l'invention ;
- la figure 4 montre un système de suivi des pressions plantaires selon l'invention.

### DESCRIPTION DÉTAILLÉE

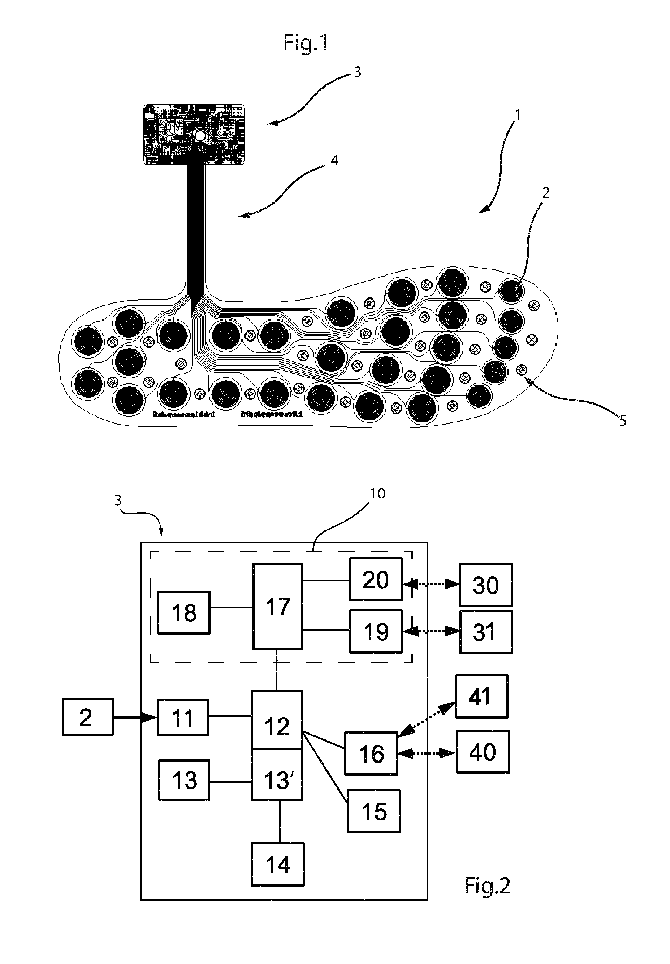

La figure 1 montre une semelle 1 selon l'invention munie d'un circuit électronique sur lequel sont implantés une pluralité de capteurs de pression 2. Le circuit électronique comprend également un module électronique 3 qui, comme représenté sur la figure 1, est de préférence disposé sur une languette 4 de la semelle de sorte que le module électronique 3 puisse être à l'extérieur d'une chaussure lorsque la semelle y est disposée. Ceci permet de disposer la semelle dans tout type de chaussure et permet avantageusement d'utiliser un chargeur inductif pour recharger la batterie du module électronique comme cela sera décrit dans la suite. L'utilisation d'une languette sur laquelle est disposé le module électronique 3 permet également de limiter l'épaisseur de la semelle. Il est évident que le module électronique 3 peut toutefois être disposé directement sur la semelle, sous la semelle, à l'intérieure de celle-ci, etc. Le module électronique 3 comprend plusieurs interfaces qui permettent de traiter les signaux envoyés par chacun des capteurs 2 et est connecté à chacun des capteurs 2 au moyen d'une piste conductrice, d'un fil ou tout autre moyen permettant de connecter électriquement un capteur 2 au module électronique 3. Lorsque le module électronique se trouve sur une languette de la semelle, comme représenté sur la figure 1, il est évident que la languette peut elle-même également être munie de pistes conductrices ou tout autre moyen permettant de connecter électriquement les capteurs de pression 2 au module électronique 3.

La semelle selon l'invention est par ailleurs munie d'une pluralité de trous traversants 5 pour permettre l'évacuation de la transpiration à travers la semelle.

Les capteurs de pression 2 sont des capteurs inductifs de forme cylindrique. Ils peuvent toutefois être de forme parallélépipédique ou en forme de cylindre elliptique. De préférence, l'épaisseur d'un capteur ne dépasse pas 2mm et son diamètre est compris entre 5mm et 20mm. Le nombre de capteurs de pression 2 implantés sur le circuit électronique de la semelle peut être compris entre 4 et 50. Ce nombre est relativement faible mais est largement suffisant pour obtenir des informations pertinentes sur les pressions élevées qui engendrent la formation des ulcères plantaires. Le fait de limiter de telle sorte le nombre de capteurs permet, entre autre, de réduire le nombre de couches du circuit électronique, rendant ainsi la semelle plus fine, et de réduire le flot de données vers le module électronique ce qui permet de limiter la consommation en énergie.

La figure 2 montre une vue schématique du module électronique 3. Le module électronique 3 est connecté à au moins un capteur 2 et comprend des moyens de mesure 11 permettant de mesurer la tension en sortie de chacun des capteurs 2. Le module électronique 3 comprend de préférence des moyens de calcul 12, par exemple un microcontrôleur, pour déterminer, en fonction des mesures de tension effectuées par les moyens de mesure 11, la pression exercée en divers points de la semelle. Le module électronique 3 comprend également des moyens d'alimentation 10 qui comprennent une batterie ou une pile rechargeable 18, une unité de charge 17 pour commander la charge de la batterie 18, un module de couplage inductif 20 pouvant être connecté par couplage inductif à un chargeur inductif 30 externe au module électronique 3 et un module de couplage filaire 19 pouvant être connecté à un chargeur 31 par l'intermédiaire d'un câble ou d'un fil. L'avantage du module de couplage inductif 20 est de permettre de recharger la batterie 18 facilement sans avoir besoin d'effectuer aucun branchement. En effet, pour recharger la batterie, l'utilisateur peut simplement laisser ses chaussures à proximité du chargeur inductif 30. L'utilisateur n'a pas à se baisser, ce qui facilite l'utilisation des semelles selon l'invention pour des personnes qui souffrent d'une mobilité réduite.

Selon une variante de l'invention, la semelle 1 peut également être munie d'un matériau piézo-électrique afin de produire de l'énergie grâce à la pression plantaire en vue de recharger la batterie 18 du module électronique 3.

Le module électronique 3 comprend également de préférence des moyens de stockage de données 14, par exemple une EEPROM ou mémoire flash. Le module électronique 3 comprend par ailleurs un module d'indication 15, comprenant par exemple une pluralité de témoins lumineux, pour indiquer l'état de charge de la batterie ou indiquer des erreurs de fonctionnement du module électronique 3. Le module électronique 3 comprend encore des moyens de communication 16 comprenant une antenne et des moyens logiciels pour émettre des données sous la forme de signaux radiofréquences vers un récepteur de signaux radiofréquences externe au module électronique 40. Les moyens de communication 16 sont également pourvus d'un connecteur et d'un logiciel pour émettre des données vers un récepteur 40 qui serait connecté au module électronique 3 au moyen d'un câble. Au sens de la présente invention, on entend par récepteur tout appareil électronique de communication qui permet de recevoir des informations (par ex. : smartphone, PC, PDA, tablette, etc.).

Le module électronique (3) comprend finalement un accéléromètre 13, pour effectuer des mesures des mouvements du module électronique 3, et un organe de commutation 13' permettant, lorsque les mesures des mouvements du module électronique 3 effectuées par l'accéléromètre montrent que le module électronique 3 n'est pas en mouvement, de faire basculer le module électronique dans un état de veille (STB), état dans lequel sa consommation d'énergie est réduite. Bien entendu, l'organe de commutation 13' permet également de faire basculer le module électronique de l'état de veille à un état actif lorsque les mesures effectuées par l'accéléromètre montrent que le module électronique est en mouvement. Ces caractéristiques permettent de limiter l'énergie consommée par le module électronique 3 et permettent donc d'économiser la batterie. Le module électronique 3 peut également comprendre un gyroscope.

En résumé, compte tenu de ses composants, le module électronique 3 est donc apte à mesurer la tension en sortie d'une pluralité de capteurs 2, déterminer sur la base de ces mesures les pressions exercées en divers points de la semelle et transmettre ces données de pression vers un ou plusieurs récepteurs distants.

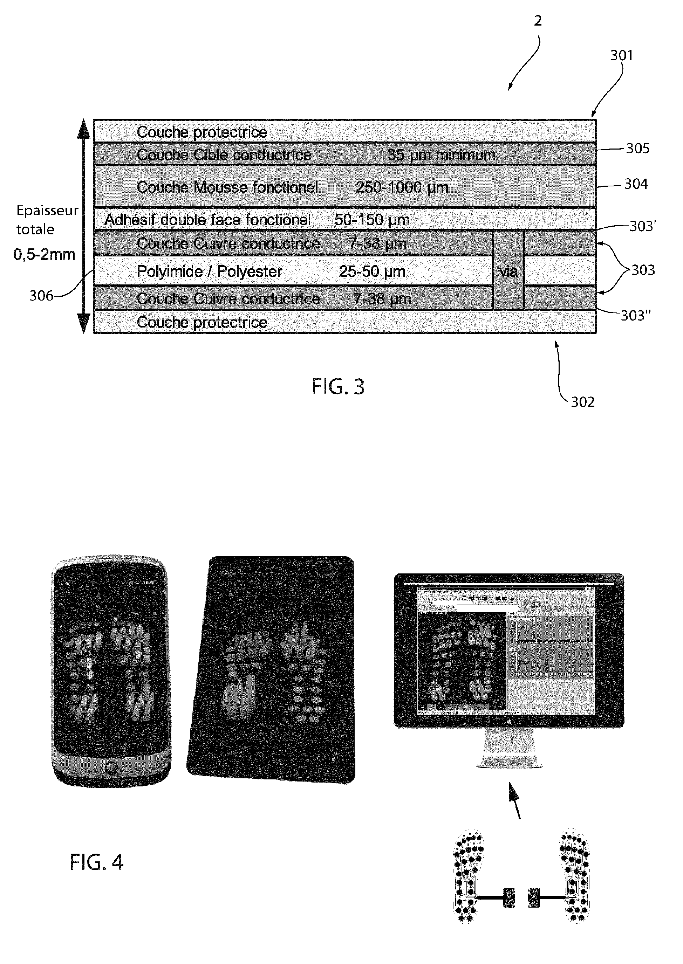

La figure 3 montre une vue selon une coupe transversale du capteur de pression selon l'invention. Comme on peut le voir, la structure d'un capteur 2 se présente sous la forme d'un « sandwich » de plusieurs couches de différents matériaux empilées les unes sur les autres. Le capteur comprend une première face 301 et une deuxième face 302 planes entre lesquelles sont disposées les couches.

Le capteur 2 est de type inductif car il comprend en particulier une bobine 303. La bobine 303 est formée de deux couches électriquement conductrices (303', 303") réalisées par exemple sous forme de deux enroulements de pistes conductrices reliés électriquement l'un à l'autre. Entre les deux couches conductrices (303', 303") est disposée une couche isolante 306 plane, réalisée dans un matériau électriquement non conducteur. La couche isolante 306 peut par exemple être constituée d'un polyimide ou d'un polyesther ou tout autre matériau électriquement isolant. L'épaisseur de la couche isolante 306 est comprise entre 25 et 100 µm.

Les pistes conductrices peuvent par exemple présenter une forme spiralée. La bobine 303 est de préférence fabriquée selon les techniques de fabrication des circuits imprimés flexible (Flexible Printed Circuit Boards ou FPCB). Chaque piste conductrice peut par exemple être en cuivre. Les enroulements des pistes conductrices sont tels que le courant électrique circule dans le même sens dans les deux enroulements. L'épaisseur de chacune des couches conductrices 303', 303" est de préférence comprise entre 7 et 38 µm. Les pistes conductrices sont reliées électriquement entre elles par exemple au travers d'un « via » traversant la couche plane 306. Un « via » se présente sous la forme d'un trou métallisé reliant les pistes conductrices entre elles.

La structure en deux couches conductrices de la bobine 303 permet de doubler le champ magnétique et ainsi d'augmenter la sensibilité du capteur 2. De plus, les deux enroulements sont isolés l'un de l'autre par la couche plane 306 et peuvent être rapprochés le plus possible pour annuler l'effet magnétique/inductance des pistes.

Le capteur comprend de plus une couche élastiquement déformable 304 réalisée en un matériau élastiquement compressible et une couche cible 305, ou cible, réalisée en un matériau électriquement conducteur. Le matériau de la couche élastiquement déformable 304 disposée entre la bobine bicouche et la cible 305 est perméable au champ magnétique. Il peut être formé de matériaux plastiques, par exemple une mousse élastomère.

Finalement, une couche de protection est disposée sur chaque face du capteur afin d'empêcher une oxydation éventuelle. Ces couches de protection ne sont toutefois pas obligatoires et leur présence dépend de l'intégration du capteur dans son milieu.

La mousse élastomère de la couche élastiquement déformable 304 est soit autocollante, soit déposée à l'aide d'un adhésif double face de transfert. Le tout est laminé sous pression sur une face. La couche cible 305 est soit déposée par jet d'une encre conductrice ou collée et laminée sur la mousse. Selon une variante d'exécution, on peut ajouter une deuxième couche élastiquement déformable et une deuxième couche cible en dessous de la bobine 303.

Le fonctionnement de la semelle selon l'invention est le suivant.

Lorsqu'un courant électrique envoyé par le module électronique 3 circule dans la bobine 303 d'un capteur, il se produit un champ magnétique qui est dévié par la couche cible 305 du capteur. Le capteur possède donc une inductance propre. Lorsqu'une pression est exercée sur le capteur, cette pression induit une compression de la couche élastiquement déformable 304 et ceci a pour conséquence de rapprocher la bobine 303 et la cible 305. Ainsi, sous l'action d'une pression positive ou négative, la bobine 303 et la cible 305 se rapprochent ou s'éloignent et ceci contribue donc à modifier le chemin du flux magnétique. Ceci a pour conséquence de faire varier l'inductance de la bobine 303. Cette inductance qui varie en fonction de la distance entre la bobine 303 et la cible 305 peut être mesurée au travers de la tension aux bornes de la bobine 303 (appelée force électromotrice), en d'autres termes aux bornes du capteur. Sur la base de la variation de la tension mesurée aux bornes du capteur par les moyens de mesure 11 du module électronique, les moyens de calcul 12 du module électronique 3 déterminent la pression exercée sur le capteur.

Ainsi, en utilisant tous les capteurs 2 de la semelle, le module électronique 3 peut déterminer les pressions exercées en divers points de la semelle. Ces données de pression peuvent ensuite être transmises par les moyens de communication 16 vers un récepteur.

La figure 4 montre schématiquement un système de suivi des pressions plantaires selon l'invention. Le dispositif comprend une ou deux semelles telles que décrites ci-dessus et un logiciel pour l'exploitation des données de pression installé sur un récepteur muni d'une interface graphique (smartphone, PC, tablette, PDA, etc.). Ces moyens logiciels permettent entre autre de présenter graphiquement sur l'interface graphique du récepteur, en utilisant les informations transmises par la ou les semelles, l'évolution des pressions plantaires exercées en divers points de la ou des semelles.

Sur l'interface graphique peut être présenté l'évolution des pressions plantaires, les mouvements et déplacements du centre de gravité, etc. Un utilisateur du dispositif, par exemple un patient souffrant de diabète et donc courant un risque d'ulcère plantaire, peut donc afficher en temps réel l'évolution des pressions plantaires générées au cours de la marche sur l'interface graphique d'un récepteur portable apte à recevoir des signaux radiofréquences sur lequel est installé le logiciel d'exploitation de données.

Un tel système de suivi permet à l'utilisateur de visualiser en temps réel l'incidence de sa marche sur les pressions plantaires pour, le cas échéant, modifier directement sa marche. Puisque le module électronique 3 comprend des moyens de stockage 14, une autre possibilité consiste à stocker les données de pression au niveau des moyens de stockage 14 du module électronique et de les télécharger quand cela est nécessaire, soit au moyen d'une interface radio soit par le biais d'une connexion par câble. Ceci permet par exemple de récupérer en une seule fois tout un historique des pressions plantaires.

La description précédente a fait état de moyens de calcul 12 présents dans le module électronique 3 permettant de déterminer les pressions en utilisant des mesures de tension. Pour s'affranchir de ces moyens de calcul au niveau du module électronique 3, on peut toutefois envisager que les moyens de calcul généralement présents dans un récepteur (smartphone, PC, tablette PC, PDA) soient utilisés et que cela soit, dans ce cas, au niveau du logiciel installé dans le récepteur que l'on détermine, en fonction des mesures de tension effectuées par les moyens de mesure 11, les pressions exercées sur la semelle. Dans ce cas, les mesures de tension effectuées par les moyens de mesure 11 du module électronique 3 seraient soit transmises directement et sans traitement préalable au récepteur, soit stockées dans le module de stockage 14 pour être transmises ultérieurement au récepteur.

## Revendications

1. Semelle (1) pour chaussure munie d'un circuit électronique comprenant une pluralité de capteurs de pression (2) connectés à un module électronique (3), le module électronique comprenant des moyens d'alimentation (10) pour alimenter les capteurs de pression en énergie, des moyens de mesure (11) pour effectuer des mesures de la tension en sortie de chacun des capteurs de pression, **caractérisée en ce que** les capteurs de pression sont des capteurs de pression inductifs.

2. Semelle selon la revendication 1, **caractérisée en ce que** le module électronique (3) comprend des moyens de calcul (12) pour déterminer, en fonction des mesures de la tension en sortie de chacun des capteurs de pression effectuées par les moyens de mesure, la pression exercée en plusieurs points de la semelle.

3. Semelle selon l'une des revendications précédentes, **caractérisée en ce que** le module électronique (3) comprend des moyens de communication (16) pour émettre des signaux radiofréquences.

4. Semelle selon l'une des revendications précédentes, **caractérisée en ce que** le module électronique (3) comprend un accéléromètre (13) pour effectuer des mesures des mouvements du module électronique (3).

5. Semelle selon la revendication 4, **caractérisée en ce que** le module électronique (3) comprend un organe de commutation (13') permettant, en utilisant les mesures des mouvements du module électronique (3) effectuées par l'accéléromètre (13), de faire basculer le module électronique (3) dans un état de veille (STB) dans lequel l'énergie consommée par le module électronique est réduite.

6. Semelle selon l'une des revendications précédentes, **caractérisée en ce que** chacun des capteurs de pression (2) comprend, empilées les unes sur les autres, au moins une couche cible (305) en un matériau électriquement conducteur, au moins une couche élastiquement déformable (304) en un matériau compressible et une bobine (303).

7. Semelle selon la revendication 6, **caractérisée en ce que** la bobine (303) comporte une première couche conductrice en un matériau électriquement conducteur (303'), une deuxième couche conductrice (303") en un matériau électriquement conducteur et une couche isolante (306) en un matériau électriquement isolant, la première couche conductrice (303') étant connectée électriquement à la deuxième couche conductrice (303") et la couche isolante étant disposée entre la première couche conductrice (303') et la deuxième couche conductrice (303").

8. Semelle selon l'une des revendications précédentes, **caractérisée en ce que** les moyens de communication (16) comprennent un connecteur et un logiciel pour connecter le module électronique (3) à un récepteur au moyen d'un câble.

9. Semelle selon l'une des revendications précédentes, **caractérisée en ce que** les moyens d'alimentation (10) comprennent une batterie rechargeable (18).

10. Semelle selon la revendication 9, **caractérisée en ce que** le module électronique (3) comprend des moyens d'indication (15) pour indiquer l'état de charge de la batterie.

11. Semelle selon l'une des revendications précédentes, **caractérisée en ce que** les moyens d'alimentation (10) comprennent un module de couplage inductif (20) pour coupler le module électronique à un chargeur inductif.

12. Semelle selon l'une des revendications précédentes, **caractérisée en ce que** les moyens d'alimentation (10) comprennent un module de couplage filaire (19) pour connecter le module électronique à un chargeur au moyen d'un câble.

13. Semelle selon l'une des revendications précédentes, **caractérisée en ce que** la semelle comprend entre 4 et 50 capteurs de pression (2).

14. Semelle selon l'une des revendications précédentes, **caractérisée en ce que** le module électronique comprend des moyens de stockage (14) pour stocker des données.

15. Semelle selon l'une des revendications précédentes, **caractérisée en ce qu'**elle comprend une pluralité de trous traversants (5).

16. Semelle selon l'une des revendications précédentes, **caractérisée en ce que** la semelle comprend une languette (4) sur laquelle est disposé le module électronique (3) de sorte que celui-ci puisse être à l'extérieur d'une chaussure lorsque la semelle y est disposée.

17. Dispositif de suivi des pressions plantaires, **caractérisé en ce que** ledit dispositif comprend une ou deux semelles selon l'une des revendications précédentes et des moyens logiciels pour présenter graphiquement sur l'interface graphique d'un récepteur, en utilisant des informations transmises par la ou les semelles, l'évolution des pressions plantaires exercées en plusieurs points de la ou des semelles.
